# EUROPEAN PATENT SPECIFICATION

(11) **EP 4 588 526 B1**
(45) Date of publication and mention of the grant of the patent: **09.12.2026**
(21) Application number: 23865025.3
(22) Date of filing: 26.06.2023
(51) Int. Cl.: A61Q 1/02, A45D 33/00, A61K 8/02

(54) **METHOD FOR PRODUCING MULTICOLORED SOLID COSMETIC**
VERFAHREN ZUR HERSTELLUNG EINES MEHRFARBIGEN FESTEN KOSMETIKUMS
PROCÉDÉ DE PRODUCTION D'UN PRODUIT COSMÉTIQUE SOLIDE MULTICOLORE

(30) Priority: 13.09.2022 JP 2022145424
(43) Date of publication of application: 23.07.2025
(73) Proprietor: Albion Co., Ltd., Tokyo, 104-0031 (JP)
(72) Inventor: MIYAMOTO Takafumi, Kumagaya-shi, Saitama 369-0108 (JP)
(74) Representative: Andrejewski - Honke Patent- und Rechtsanwälte Partnerschaft mbB
(86) International application number: PCT/JP2023/023666
(87) International publication number: WO 2024/057663

(56) References cited:
- JP-A- 2013 079 202
- JP-A- 2013 079 202
- JP-A- H0 482 818
- JP-A- S62 145 008
- JP-A- S62 145 008

## Description

### Technical Field

The present invention relates to a method for producing a multicolored solid cosmetic.

### Background Art

In recent years, a multicolored solid cosmetic is provided in which solid cosmetics of a plurality of colors are loaded directly adjacent to one another in a cosmetic dish without a partition.

In the related art, as a method for producing such a multicolored solid cosmetic, for example, a method is proposed in which single-color rod-shaped cosmetic is molded for several colors, these are combined to form a multicolored rod-shaped cosmetic into a small diameter using a piston-type extrusion molding machine, which are then cut in a direction perpendicular to an axis to obtain a multicolored plate-shaped solid cosmetic (see PTL 1), and a method is proposed in which a multicolored cosmetic bulk (a rod-shaped molded material with a powder cosmetic), which is made by molding a powder cosmetic into a rod shape and having the powder cosmetic adhered to a surface of a rod-shaped molded material, is accommodated in a container from which a content can be extruded, extruded in a predetermined amount in a long axis direction of the rod-shaped molded material, and cut in a direction perpendicular to the long axis of the rod-shaped molded material to obtain the multicolored solid cosmetic (see PTL 2).

### Citation List

### Patent Literature

PTL 1: JPS63-5368B
PTL 2: JP2013-79202A

### Summary of Invention

### Technical Problem

However, according to the method described in PTL 1, since the piston-type extrusion molding machine is used for molding a multicolored rod-shaped cosmetic, it is necessary to use a cosmetic bulk having a degree of softness as to be extruded by the extrusion molding machine. Similarly, according to the method described in PTL 2, since the multicolored cosmetic bulk is cut by being extruded by the predetermined amount by the container capable of extruding the content, it is necessary to use a cosmetic bulk having a degree of softness as to be extruded by the container. As a result, according to the methods in the related art, a range of selection of a cosmetic bulk that can be used may be limited.

On the other hand, according to the method described in PTL 2, since the predetermined amount of the content is extruded and cut using the container capable of extruding the content during cutting of the multicolored cosmetic bulk, the cosmetic bulk may be unintentionally hardened and altered in quality by the extrusion during cutting.

The invention has been made in view of the above points, and an object of the invention is to provide a method for producing a multicolored solid cosmetic that has a wide range of selection of a cosmetic bulk used for production and which can prevent hardness of the cosmetic bulk from being unintentionally altered in quality.

### Solution to Problem

In order to achieve the above object, a method for producing a multicolored solid cosmetic according to the invention includes: a rod-shaped molded body acquisition step of obtaining a multicolored rod-shaped molded body by adhering, to a periphery of a rod-shaped molded body obtained by molding a powder cosmetic into a rod shape, a powder cosmetic having a color different from a color of the rod-shaped molded body; a compression step of obtaining, by compressing the multicolored rod-shaped molded body in an amount of two or more disposed with central axes thereof parallel to each other in a container formed by uniting two or more split molds, a multicolored cosmetic bulk having a shape corresponding to an internal shape of the container; and a cutting step of cutting the multicolored cosmetic bulk taken out from the container by separating the two or more split molds in a direction orthogonal to the central axis of the rod-shaped molded body.

In the method for producing a multicolored solid cosmetic according to the invention, at least one end side of the container in a longitudinal direction is formed with a side wall with which a movement in the longitudinal direction of the container is restricted, and the cutting step is performed in a state in which the multicolored cosmetic bulk is placed on a placement surface.

In the method for producing a multicolored solid cosmetic according to the invention, the container has a first split mold and a second split mold divided along the longitudinal direction of the container, and in the compression step, the first split mold and the second split mold are united and closed to compress the two or more multicolored rod-shaped molded bodies disposed in the first split mold and the second split mold.

In the method for producing a multicolored solid cosmetic according to the invention, the container has a first split mold having an opening along the longitudinal direction of the container and a second split mold configured to close the opening, and in the compression step, by pressing with the second split mold from the opening of the first split mold, the two or more multicolored rod-shaped molded bodies disposed in the first split mold are compressed.

In the method for producing a multicolored solid cosmetic according to the invention, the container has a first split mold and a second split mold divided along a longitudinal direction of the container, one end side of the container in the longitudinal direction is formed with a side wall with which a movement in the longitudinal direction of the container is restricted, the other end side of the container in the longitudinal direction has an opening, and in the compression step, in a state in which the first split mold and the second split mold are united, the two or more multicolored rod-shaped molded bodies in the container are compressed by being pressed by a pressing member from the opening.

In the method for producing a multicolored solid cosmetic according to the invention, the container has a first split mold and a second split mold in which a hemispherical cavity is formed, and in the compression step, the first split mold and the second split mold are united and closed to compress the two or more multicolored rod-shaped molded bodies disposed in the first split mold and the second split mold into a spherical shape.

In addition, a method for producing a multicolored solid cosmetic includes: a rod-shaped molded body acquisition step of obtaining, in each desired color, a rod-shaped molded body formed by molding a powder cosmetic into a rod shape; a compression step of obtaining, by compressing the rod-shaped molded body in an amount of two or more disposed with central axes thereof parallel to each other in a container formed by uniting two or more split molds, a multicolored cosmetic bulk having a shape corresponding to an internal shape of the container; and a cutting step of cutting the multicolored cosmetic bulk taken out from the container by separating the two or more split molds in a direction orthogonal to the central axis of the rod-shaped molded body.

In the method for producing a multicolored solid cosmetic according to the invention, the cutting step is performed in a state in which the multicolored cosmetic bulk is placed on a placement surface.

### Advantageous Effects of Invention

According to the invention, a method for producing a multicolored solid cosmetic can be provided, which has a wide range of selection of a cosmetic bulk used for production and which can prevent hardness of the cosmetic bulk from being unintentionally altered in quality.

### Brief Description of Drawings

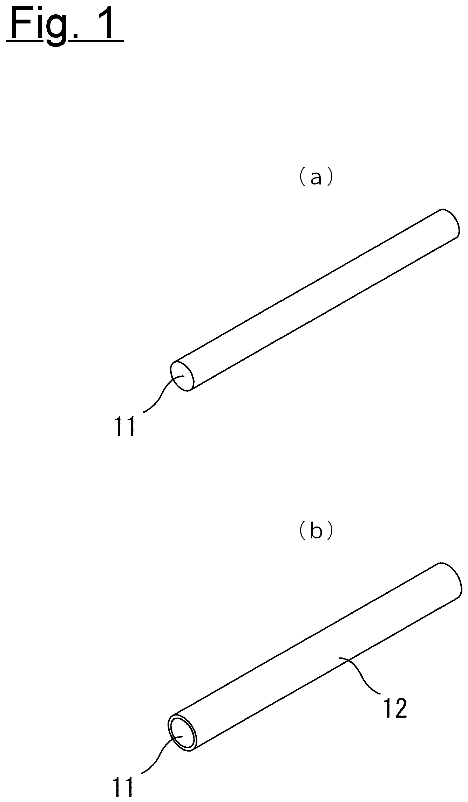

[FIG. 1] FIG. 1 shows diagrams showing a method for producing a multicolored solid cosmetic according to an embodiment of the invention, where (a) is a perspective view of a rod-shaped molded body, and (b) is a perspective view of a multicolored rod-shaped molded body.

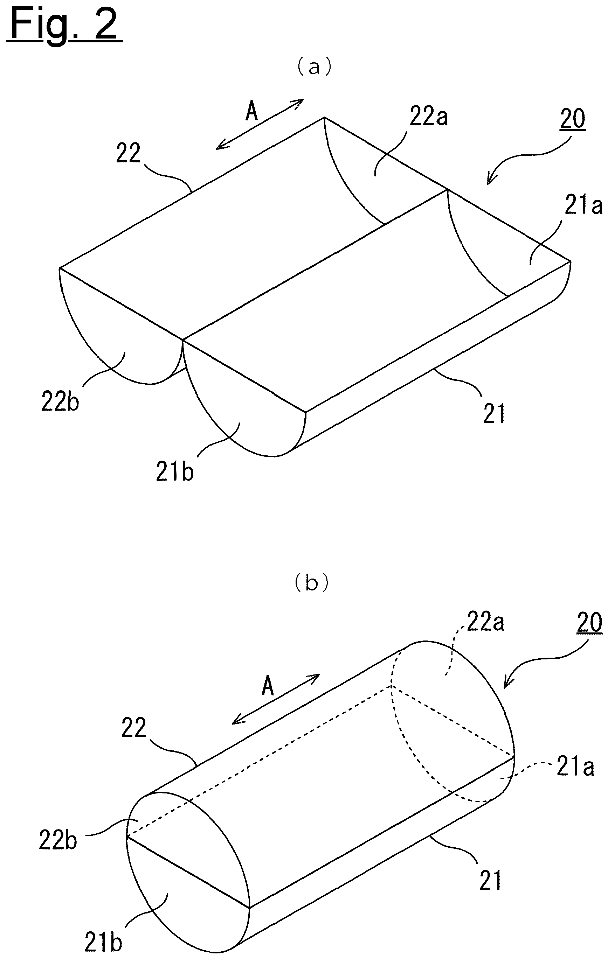

[FIG. 2] FIG. 2 shows diagrams showing a container used in the method for producing the multicolored solid cosmetic according to the embodiment of the invention, where (a) is a perspective view of the container in a state in which split molds are separated, and (b) is a perspective view of the container in a state in which the split molds are united.

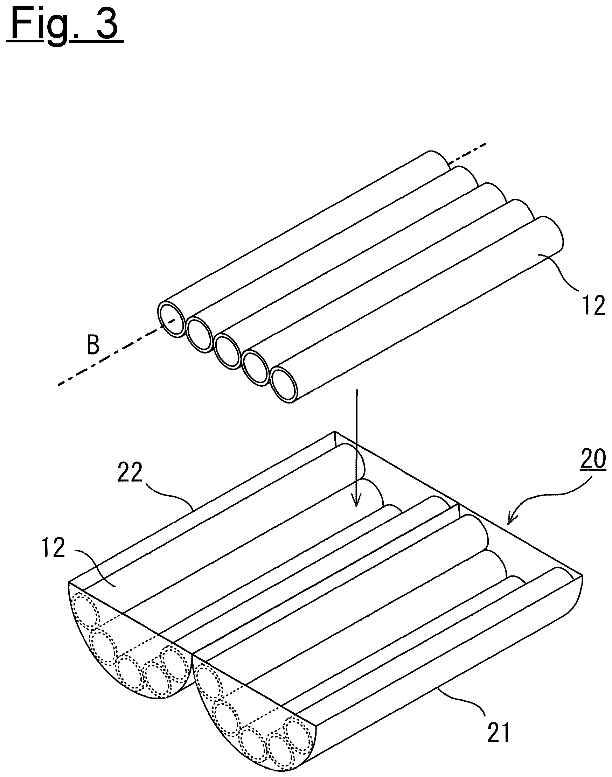

[FIG. 3] FIG. 3 is a perspective view showing an accommodation step in the method for producing the multicolored solid cosmetic according to the embodiment of the invention.

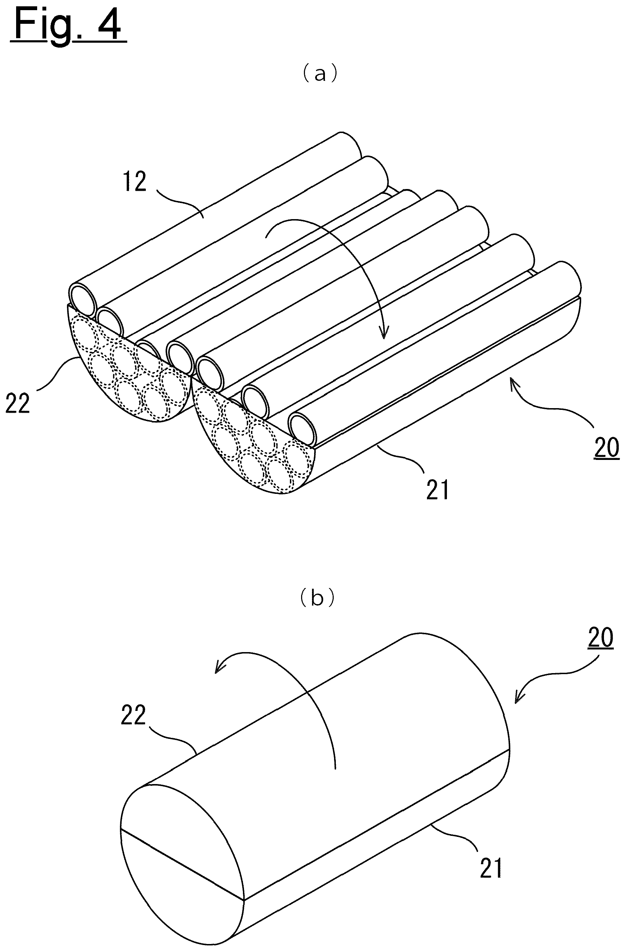

[FIG. 4] FIG. 4 is a perspective view showing a compression step in the method for producing the multicolored solid cosmetic according to the embodiment of the invention.

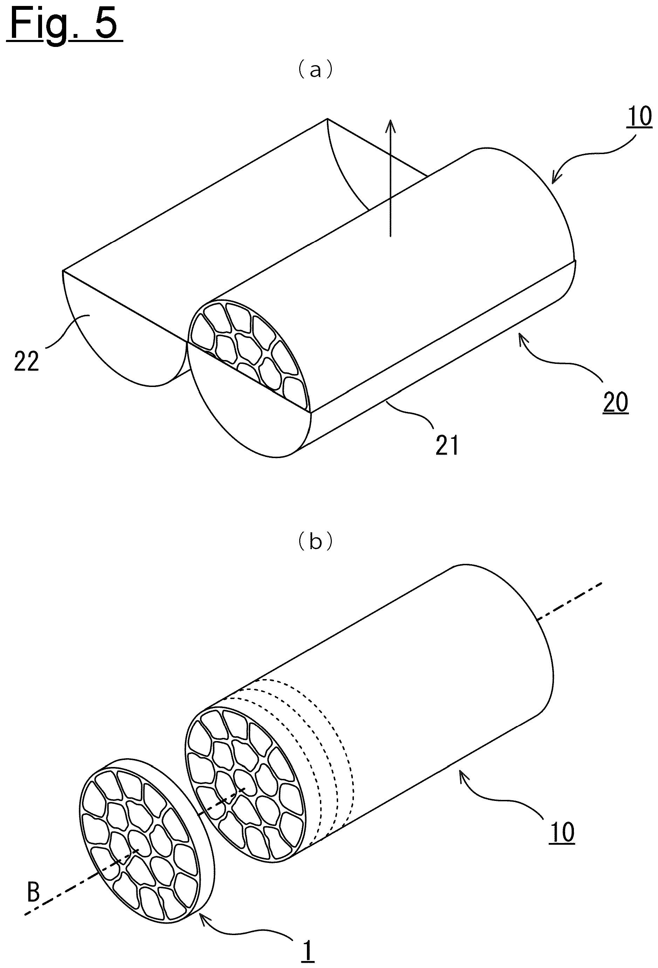

[FIG. 5] FIG. 5 shows diagrams showing the method for producing the multicolored solid cosmetic according to the embodiment of the invention, where (a) is a perspective view showing a taking-out step of a multicolored cosmetic bulk, and (b) is a perspective view showing a cutting step of the multicolored cosmetic bulk.

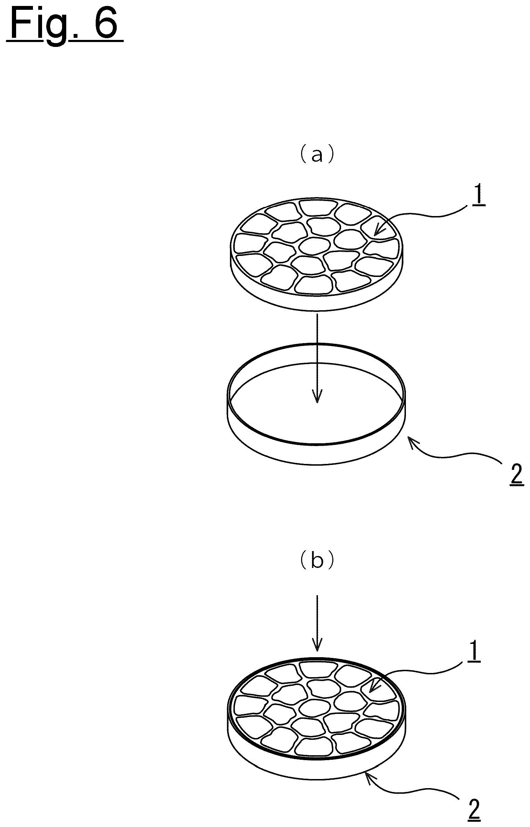

[FIG. 6] FIG. 6 shows diagrams showing the method for producing the multicolored solid cosmetic according to the embodiment of the invention, where (a) is a perspective view showing a loading step of the multicolored solid cosmetic, and (b) is a perspective view showing a pressing step of the multicolored solid cosmetic.

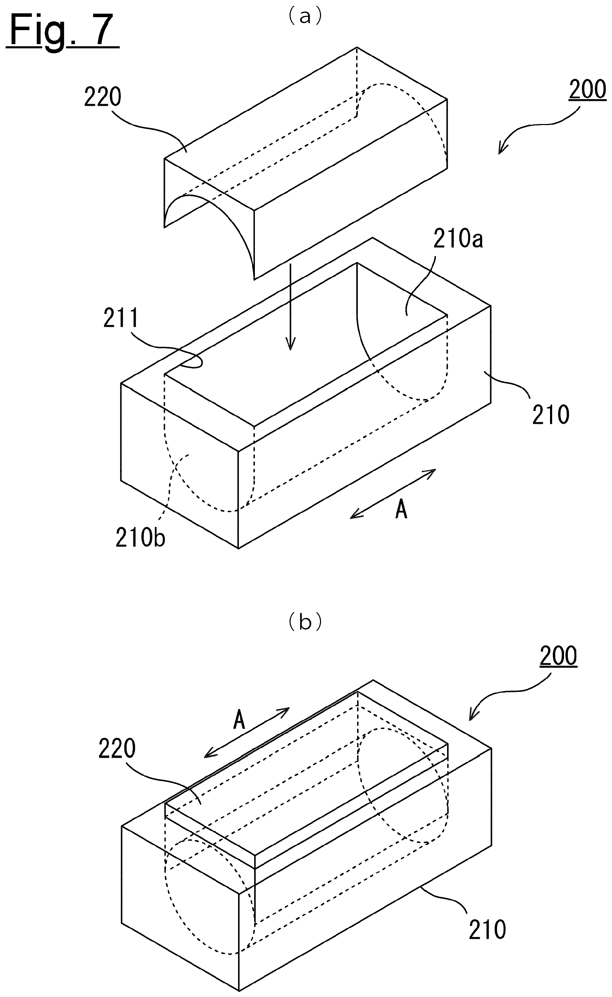

[FIG. 7] FIG. 7 shows perspective views of a container used in a method for producing a multicolored solid cosmetic according to Modification 2 of the embodiment of the invention, where (a) is a perspective view of the container in a state in which split molds are separated, and (b) is a perspective view of the container in a state in which the split molds are united.

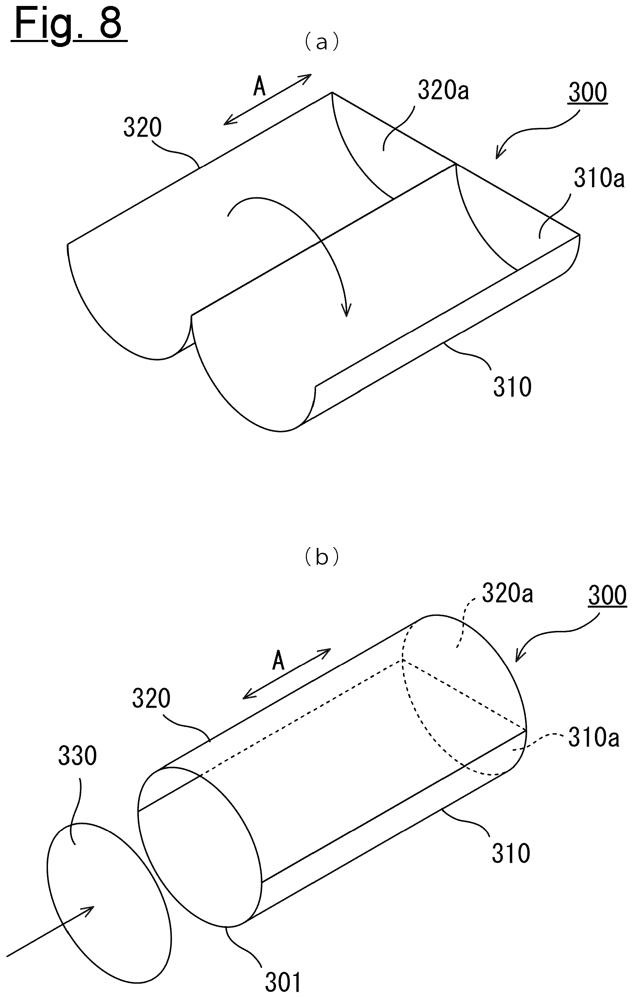

[FIG. 8] FIG. 8 shows perspective views of a container used in a method for producing a multicolored solid cosmetic according to Modification 3 of the embodiment of the invention, where (a) is a perspective view of the container in a state in which split molds are separated, and (b) is a perspective view of the container in a state in which the split molds are united.

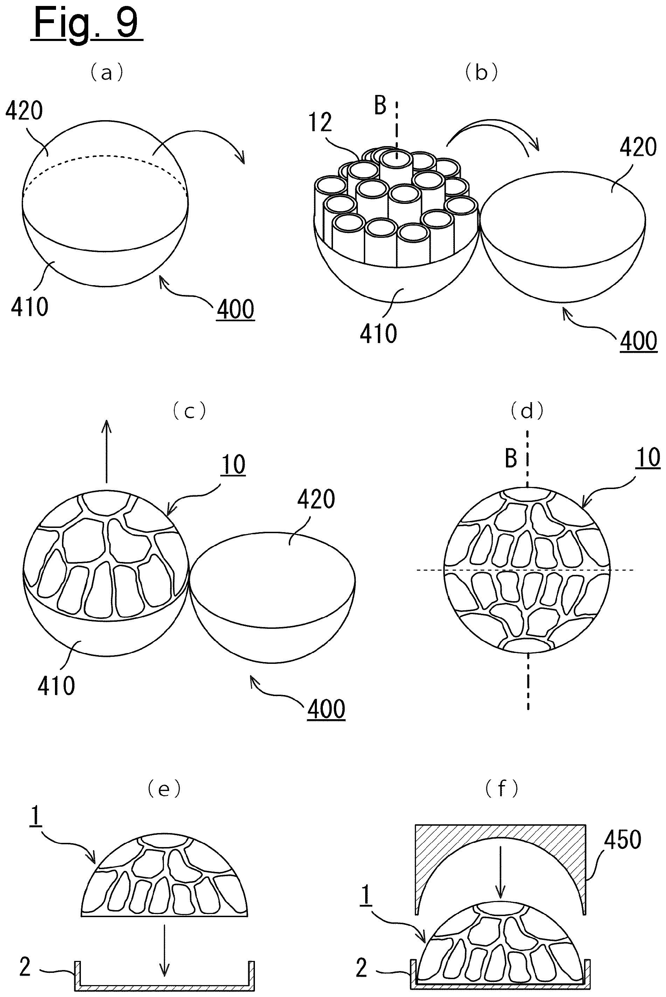

[FIG. 9] FIG. 9 shows diagrams showing a method for producing a multicolored solid cosmetic according to Modification 4 of the embodiment of the invention, where (a) is a perspective view of a container in a state in which split molds are united, (b) is a perspective view showing an accommodation step and a compression step, (c) is a perspective view showing a taking-out step of a multicolored cosmetic bulk, (d) is a front view showing a cutting step of the multicolored cosmetic bulk, (e) is a view showing a loading step of the multicolored solid cosmetic, and (f) is a view showing a pressing step of the multicolored solid cosmetic.

### Description of Embodiments

A method for producing a multicolored solid cosmetic according to the invention includes: a rod-shaped molded body acquisition step of obtaining a multicolored rod-shaped molded body by adhering, to a periphery of a rod-shaped molded body obtained by molding a powder cosmetic into a rod shape, a powder cosmetic having a color different from a color of the rod-shaped molded body; a compression step of obtaining, by compressing the multicolored rod-shaped molded body in an amount of two or more disposed with central axes thereof parallel to each other in a container formed by uniting two or more split molds, a multicolored cosmetic bulk having a shape corresponding to an internal shape of the container; and a cutting step of cutting the multicolored cosmetic bulk taken out from the container by separating the two or more split molds in a direction orthogonal to the central axis of the rod-shaped molded body.

Hereinafter, the method for producing the multicolored solid cosmetic according to an embodiment of the invention will be described with reference to the drawings. The method for producing the multicolored solid cosmetic according to the invention is particularly suitable for producing face powders such as blushers, eye shadows, and other face powders, that is, a multicolored solid powder cosmetic for make-up.

### [Rod-Shaped Molded Body Acquisition Step]

With reference to FIG. 1, a rod-shaped molded body acquisition step in the method for producing the multicolored solid cosmetic according to the embodiment will be described. FIG. 1 shows diagrams showing the method for producing the multicolored solid cosmetic according to the embodiment of the invention, where (a) is a perspective view of a rod-shaped molded body, and (b) is a perspective view of a multicolored rod-shaped molded body.

The rod-shaped molded body acquisition step in the method for producing the multicolored solid cosmetic according to the embodiment is a step of obtaining a multicolored rod-shaped molded body by adhering, to a periphery of a rod-shaped molded body obtained by molding a powder cosmetic into a rod shape, a powder cosmetic having a color different from that of the rod-shaped molded body.

As shown in (a) of FIG. 1, two or more rod-shaped molded bodies 11 are obtained by molding the powder cosmetic into the rod shape. As a method for molding the rod-shaped molded body 11, for example, an injection molding method for extruding a single-color cosmetic bulk in a slurry state or a clay state into a screw extrusion hopper, and a method for press-molding using a press mold can be used.

The powder cosmetic used for molding the rod-shaped molded body 11 may be any material that contains powder and can be molded into a rod shape, and a form during molding is not particularly limited. For example, a powder cosmetic in the form of a powder, a granule, an aggregate, a clay, a slurry, or a mixture thereof can be used to mold the rod-shaped molded body 11. When preparing the powder cosmetics in these forms, it is possible to adjust a composition by adding an appropriate amount of a known volatile solvent, if necessary.

Each of the two or more rod-shaped molded bodies 11 may be molded from powder cosmetics of the same color, or may be molded from powder cosmetics of mutually different colors.

Hardness of the rod-shaped molded body 11 is not limited as long as it is adjusted appropriately depending on usability and the like as a multicolored solid cosmetic 1, whereas it may be preferable if the rod-shaped molded body has hardness that allows it to maintain a molded shape when left still, as it improves workability.

A shape of the rod-shaped molded body 11 is not particularly limited, and can be appropriately selected so as to obtain a desired pattern when a multicolored cosmetic bulk 10 including the rod-shaped molded body 11 is cut. Therefore, the rod-shaped molded body 11 may have a cylindrical shape as shown in (a) of FIG. 1, or may have another polygonal prism shape.

Subsequently, as shown in (b) of FIG. 1, a powder cosmetic is adhered to a periphery of the rod-shaped molded body 11, thereby obtaining a multicolored rod-shaped molded body 12. As the powder cosmetic adhered to the periphery of the rod-shaped molded body 11, it is preferable to use a powder cosmetic having a color different from that of the powder cosmetic used for molding the rod-shaped molded body 11. A mesh pattern of a color different from that of the rod-shaped molded body 11 can be made to appear on the multicolored solid cosmetic 1 obtained through a step to be described later using the multicolored rod-shaped molded body 12 (see (b) of FIG. 5 and FIG. 6). Accordingly, it is possible to provide the multicolored solid cosmetic 1 which can be taken up with a puff and used for make up so that powder cosmetics of different colors, which are at least the powder cosmetic forming the rod-shaped molded body 11 and the powder cosmetic adhering to the periphery of the rod-shaped molded body 11, are blended.

The method for adhering the powder cosmetic to the periphery of the rod-shaped molded body 11 is not particularly limited. As an example, a method in which the rod-shaped molded body 11 is rolled on a powder cosmetic put in a flat tray to adhere the powder cosmetic to the periphery of the rod-shaped molded body 11, a method in which the rod-shaped molded body 11 is placed on a powder cosmetic scattered on a belt conveyor and the belt conveyor is driven while pressing the rod-shaped molded body 11 from above by a pressing member to adhere the powder cosmetic to the periphery of the rod-shaped molded body 11, or the like may be used. Accordingly, a substantially uniform powder cosmetic layer can be formed on the periphery of the rod-shaped molded body 11.

A region of the rod-shaped molded body 11 to which the powder cosmetic is adhered is freely set, and as shown in (b) of FIG. 1, the powder cosmetic may be adhered only to a side surface of the rod-shaped molded body 11, or may be adhered to the entire surface including both end surfaces (bottom surfaces) in a longitudinal direction of the rod-shaped molded body 11, and is not particularly limited.

A thickness of the powder cosmetic layer formed on the periphery of the rod-shaped molded body 11 may be appropriately adjusted according to a desired width of the mesh pattern to appear on the multicolored solid cosmetic 1.

In order to the obtain a multicolored solid cosmetic with a more complex pattern, two or more powder cosmetic layers may be formed on the rod-shaped molded body 11, and in that case, the second powder cosmetic layer may be formed of a powder cosmetic of a color different from the color of the powder cosmetic used for the first powder cosmetic layer.

### [Accommodation Step and Compression Step]

The accommodation step and the compression step in the method for producing the multicolored solid cosmetic according to the embodiment will be described with reference to FIGS. 2 to 4. FIG. 2 shows diagrams showing a container used in the method for producing the multicolored solid cosmetic according to the embodiment of the invention, where (a) is a perspective view of the container in a state in which split molds are separated, (b) is a perspective view of the container in a state in which the split molds are united. FIG. 3 is a perspective view showing the accommodation step in the method for producing the multicolored solid cosmetic according to the embodiment of the invention. FIG. 4 is a perspective view showing the compression step in the method for producing the multicolored solid cosmetic according to the embodiment of the invention.

The accommodation step in the method for producing the multicolored solid cosmetic according to the embodiment is a step of disposing and accommodating two or more multicolored rod-shaped molded bodies in a container with their central axes parallel to each other. In addition, the compression step in the method for producing the multicolored solid cosmetic according to the embodiment is a step of obtaining a multicolored cosmetic bulk having a shape corresponding to an internal shape of a container by compressing the multicolored rod-shaped molded body in an amount of two or more disposed with their central axes parallel to each other in the container implemented by combining two or more split molds.

As shown in FIG. 3, the two or more multicolored rod-shaped molded bodies 12 are disposed and accommodated in a container 20 with their central axes B parallel to one another.

The container according to the embodiment includes a first split mold and a second split mold that are split along the longitudinal direction of the container. As an example, as shown in (a) of FIG. 2, the container 20 according to the embodiment includes a first split mold 21 and a second split mold 22, each of which has an open upper surface and is formed by dividing the container 20 in the cylindrical shape into two equal parts along a longitudinal direction A. Further, as shown in (b) of FIG. 2, by uniting and closing the first split mold 21 and the second split mold 22, a cylindrical internal shape (accommodation space) is formed. Accordingly, as shown in (a) of FIG. 3 to be described later, the multicolored rod-shaped molded body 12 can be accommodated from an opened upper surface side of each of the first split mold 21 and the second split mold 22.

The container according to the embodiment includes a side wall with which a movement in the longitudinal direction of the container is restricted at least on one end side of the container in the longitudinal direction. As an example, the container 20 includes side walls 21a, 21b, 22a, and 22b with which a movement in the longitudinal direction A of the container 20 is restricted on both end sides in the longitudinal direction A. For example, as shown in FIG. 2, the side walls 21a, 21b, 22a, and 22b of the container 20 may be fixedly provided, and the container 20 does not need to have a structure for extruding a content in the accommodation space.

The container 20 according to the embodiment is described as having a cylindrical internal shape (accommodation space) when the split molds are united (see (b) of FIG. 2), whereas the internal shape is not particularly limited, and can be changed as appropriate depending on an external shape of the multicolored solid cosmetic 1 to be finally produced.

A material of the container 20 is not particularly limited as long as it can accommodate the two or more multicolored rod-shaped molded bodies 12, and from the viewpoint of workability and hygiene, it is preferable to use a resin or a metal material.

From the viewpoint of making a mesh pattern appear on the multicolored solid cosmetic 1, as shown in FIG. 3, it is preferable to dispose and accommodate the two or more multicolored rod-shaped molded bodies 12 in the container 20. The number and arrangement of the multicolored rod-shaped molded bodies 12 to be accommodated may be appropriately adjusted according to a desired pattern to appear on the multicolored solid cosmetic 1.

The multicolored rod-shaped molded body 12 is preferably disposed in the container 20 so that a desired pattern appears on the multicolored solid cosmetic 1 obtained through the steps to be described later. At this time, as necessary, an appropriate amount of a powder cosmetic having the quality same as that of the powder cosmetic adhered to the periphery of the rod-shaped molded body 11 may be replenished in a gap between the container 20 and the multicolored rod-shaped molded body 12 or a gap between two or more multicolored rod-shaped molded bodies 12. Accordingly, the multicolored rod-shaped molded body 12 can be disposed at a desired position in the container 20, and can be reflected on the pattern formed on a surface of the multicolored solid cosmetic 1.

The two or more multicolored rod-shaped molded bodies 12 disposed in the container 20 may have the rod-shaped molded bodies 11 of the same color or may have the rod-shaped molded bodies 11 of different colors.

In order to easily take out the multicolored cosmetic bulk 10 to be described later from the container 20, when the two or more multicolored rod-shaped molded bodies 12 are disposed in the container 20, a nonwoven fabric or the like can be previously laid on the first split mold 21 and the second split mold 22, which is preferable.

As shown in (a) of FIG. 4, after the two or more multicolored rod-shaped molded bodies 12 are disposed and accommodated in the container 20 (each of the first split mold 21 and the second split mold 22), the second split mold 22 is united with the first split mold 21 in an arrow direction, and as shown in (b) of FIG. 4, the first split mold 21 and the second split mold 22 are completely closed to compress the two or more multicolored rod-shaped molded bodies 12 disposed in the first split mold 21 and the second split mold 22.

Accordingly, the two or more multicolored rod-shaped molded bodies 12 are compressed into a shape corresponding to the internal shape of the container 20 (a cylindrical shape in the embodiment), so that a density is made more uniform, and the multicolored cosmetic bulk 10 is molded in the container 20.

### [Cutting Step]

A cutting step in the method for producing the multicolored solid cosmetic according to the embodiment will be described with reference to FIG. 5. FIG. 5 shows diagrams showing the method for producing the multicolored solid cosmetic according to the embodiment of the invention, where (a) is a perspective view showing a taking-out step of the multicolored cosmetic bulk, and (b) is a perspective view showing a cutting step of the multicolored cosmetic bulk.

The cutting step in the method for producing the multicolored solid cosmetic according to the embodiment is a step of cutting a multicolored cosmetic bulk, which is taken out from a container by separating two or more split molds, in a predetermined thickness in a direction orthogonal to the central axis of the multicolored cosmetic bulk in a state of being placed on a placement surface.

After the compression step, as shown in (a) of FIG. 5, the first split mold 21 and the second split mold 22 of the container 20 are separated, and the multicolored cosmetic bulk 10 molded in the container 20 is taken out. The multicolored cosmetic bulk 10 includes the compressed rod-shaped molded body 11 and a powder cosmetic (which is adhered to the periphery of the rod-shaped molded body 11 and/or which is replenished in the gap) which is compressed and integrated. In addition, the multicolored cosmetic bulk 10 can be uniformly formed from one end side to the other end side in a direction of the central axis (see (b) of FIG. 5) through the respective steps described above.

As shown in (b) of FIG. 5, the multicolored cosmetic bulk 10 taken out from the container 20 is cut into pieces of a predetermined thickness in a direction perpendicular to the central axis B of at least one of the rod-shaped molded bodies 11 contained in the multicolored cosmetic bulk 10, thereby obtaining the multicolored solid cosmetic 1.

In order to prevent the hardness of the multicolored solid cosmetic 1 from being unintentionally altered in quality, it is preferable that the multicolored cosmetic bulk 10 is placed on a placement surface (not shown) at the time of cutting and a unit for pressing the periphery of the multicolored cosmetic bulk 10 is not used as much as possible. The placement surface on which the multicolored cosmetic bulk 10 is placed in order to cut the multicolored cosmetic bulk 10 is not particularly limited, and may be, for example, an upper surface of a pedestal which is fixedly installed or an upper surface of a belt conveyor which is possible. The placement surface is not limited to a flat surface, and may be provided with one or more grooves through which a cutting edge of a cutting unit can be inserted, or may be provided with a shape for preventing rolling or the like of the multicolored cosmetic bulk 10.

The cutting unit is preferably a unit capable of cutting the open multicolored solid cosmetic 1 completely taken out from the container 20 without causing deformation of the shape, and more specifically, an ultrasonic cutter is preferable. Of course, other cutting units may be used depending on the hardness of the multicolored solid cosmetic 1.

The predetermined thickness is appropriately set according to a depth or the like of a cosmetic dish 2 (see FIG. 6) to be described later.

### [Pressing Step]

A pressing step in the method for producing the multicolored solid cosmetic according to the embodiment will be described with reference to FIG. 6. FIG. 6 shows diagrams showing the method for producing the multicolored solid cosmetic according to the embodiment of the invention, where (a) is a perspective view showing a loading step of the multicolored solid cosmetic, and (b) is a perspective view showing a pressing step of the multicolored solid cosmetic.

As shown in (a) of FIG. 6, the multicolored solid cosmetic 1 is loaded into the cosmetic dish 2. Thereafter, the multicolored solid cosmetic 1 is subjected to a press process from an opening side (an arrow direction in (b) of FIG. 6) of the cosmetic dish 2 by a known press device (not shown).

As described above, the method for producing the multicolored solid cosmetic according to the embodiment can efficiently produce the multicolored solid cosmetic 1 having a uniform pattern from the surface to the bottom surfaces, and also has the following effects.

In the molding (compression step) of the multicolored cosmetic bulk according to the embodiment, the multicolored cosmetic bulk is compressed and molded using a container formed of two or more split molds without using a piston-type extrusion molding machine as in the related art, and since the multicolored cosmetic bulk is taken out from the container by separating the split molds, it is possible to use a hard cosmetic bulk that cannot be extruded and molded using the extrusion molding machine in the related art. Similarly, in the cutting step of the multicolored solid cosmetic according to the embodiment, instead of extruding a content by a predetermined amount using a container that can extrude the content and cutting the content as in the related art, since the content is completely removed from the container and cut while being placed on the placement surface, it is also possible to use a hard cosmetic bulk that cannot be extruded using the extrusion container in the related art. As a result, the method for producing the multicolored solid cosmetic according to the embodiment has an effect of widening a range of selection of a cosmetic bulk that can be used.

In addition, in the cutting step of the multicolored cosmetic bulk according to the embodiment, instead of extruding a content by a predetermined amount using a container that can extrude the content and cutting the content as in the related art, since the content is cut in an open state after being completely removed from the container and placed on the placement surface, an effect of avoiding unintentional alternation in quality in the hardness of the multicolored cosmetic bulk during the production step is obtained.

Hereinafter, modifications of the above embodiment will be described.

### [Modification 1]

In the above-described embodiment, the method for producing the multicolored solid cosmetic 1 in which the mesh pattern appears by molding the multicolored cosmetic bulk 10 (see FIG. 5) using the multicolored rod-shaped molded body 12 (see (b) of FIG. 1) in which the powder cosmetic is adhered to the periphery of the rod-shaped molded body 11 is described. The invention is not limited thereto, and may be a method for producing a multicolored solid cosmetic including a rod-shaped molded body acquisition step of obtaining, in each desired color, a rod-shaped molded body (see (a) of FIG. 1) formed by molding a powder cosmetic into a rod shape, a compression step of obtaining, by compressing the rod-shaped molded body in an amount of two or more disposed with central axes thereof parallel to each other in a container formed by uniting two or more split molds, a multicolored cosmetic bulk having a shape corresponding to an internal shape of the container, and a cutting step of cutting the multicolored cosmetic bulk taken out from the container by separating the two or more split molds into a predetermined thickness in a direction orthogonal to the central axis of the rod-shaped molded body. The same applies to modifications to be described later.

According to Modification 1, it is also possible to provide a multicolored solid cosmetic in which solid cosmetics of a plurality of colors are loaded in a state of being directly adjacent to each other in a cosmetic dish without partition, while obtaining the effects same as those of the above-described embodiment.

### [Modification 2]

Hereinafter, Modification 2 of the above embodiment will be described with reference to FIG. 7. FIG. 7 shows perspective views of a container used in a method for producing a multicolored solid cosmetic according to Modification 2 of the embodiment of the invention, where (a) is a perspective view of the container in a state in which split molds are separated, and (b) is a perspective view of the container in a state in which the split molds are united.

As shown in (a) of FIG. 2, the container 20 according to the embodiment is described as including the first split mold 21 and the second split mold 22, each of which has an open upper surface and is formed by dividing the container 20 in the cylindrical shape into two equal parts along the longitudinal direction A. The invention is not limited thereto, and as shown in (a) of FIG. 7, a container 200 may include a first split mold 210 having an opening 211 along the longitudinal direction A of the container 200, and a second split mold 220 that closes the opening 211, and the compression step may include compression of the two or more multicolored rod-shaped molded bodies 12 disposed in the first split mold 210 by pressing the second split mold 220 from the opening 211 of the first split mold 210.

In the container 200 according to Modification 2, the two or more multicolored rod-shaped molded bodies 12 to be accommodated are disposed and accommodated in the first split mold 210, and then closed and compressed by the second split mold 220. As shown in (b) of FIG. 7, in the container 200 according to Modification 2, when the first split mold 210 is closed by the second split mold 220, a cylindrical internal shape (accommodation space) is formed by internal shapes of the first split mold 210 and the second split mold 220.

According to Modification 2, it is also possible to provide a multicolored solid cosmetic in which solid cosmetics of a plurality of colors are loaded in a state of being directly adjacent to each other in a cosmetic dish without partition, while obtaining the effects same as those of the above-described embodiment.

### [Modification 3]

Hereinafter, Modification 3 of the above embodiment will be described with reference to FIG. 8. FIG. 8 shows perspective views of a container used in a method for producing a multicolored solid cosmetic according to Modification 3 of the embodiment of the invention, where (a) is a perspective view of the container in a state in which split molds are separated, and (b) is a perspective view of the container in a state in which the split molds are united.

As shown in FIG. 2, the container 20 according to the above embodiment is described as including the side walls 21a, 21b, 22a, and 22b with which a movement in the longitudinal direction A of the container 20 is restricted on both end sides in the longitudinal direction A. The invention is not limited thereto, and at least one end side in the longitudinal direction of the container may be implemented by a side wall with which a movement in the longitudinal direction of the container is restricted. As an example, as shown in (a) of FIG. 8, a container 300 according to Modification 3 has a first split mold 310 and a second split mold 320 which are formed by dividing the container 300 having a bottomed cylindrical shape into two equal parts along the longitudinal direction A and whose upper surfaces are open, has side walls 310a and 320a with which a movement in the longitudinal direction A is restricted only on one end side in the longitudinal direction A, and has an opening 301 without a side wall on the other end side in the longitudinal direction A, as shown in (b) of FIG. 8. As shown in FIG. 8, the side walls 310a and 320a of the container 300 are fixedly provided as an example, and the container 300 does not require a structure for extruding a content in the accommodation space.

In the accommodation step in the case of using the container 300, it is also possible to put the multicolored rod-shaped molded body 12 into the container 300 from the opening 301 of the container 300 formed into a bottomed cylindrical shape by uniting the first split mold 310 and the second split mold 320. At this time, a partition member for disposing the multicolored rod-shaped molded body 12 may be put in the container 300 in advance, and the multicolored rod-shaped molded body 12 may be put in a space partitioned by the partition member. Accordingly, reproducibility of a pattern appearing on the multicolored solid cosmetic 1 can be enhanced.

In the compression step in the case of using the container 300, the two or more multicolored rod-shaped molded bodies 12 disposed in the container 300 are compressed by pressing the container 300 with a pressing member 330 from the opening 301 of the container 300 in which the multicolored rod-shaped molded bodies 12 are accommodated. When the partition member is used in the accommodation step, the partition member is removed before the compression step.

According to Modification 3, it is also possible to provide a multicolored solid cosmetic in which solid cosmetics of a plurality of colors are loaded in a state of being directly adjacent to each other in a cosmetic dish without partition, while obtaining the effects same as those of the above-described embodiment.

### [Modification 4]

Hereinafter, Modification 4 of the above embodiment will be described with reference to FIG. 9. FIG. 9 shows diagrams showing a method for producing a multicolored solid cosmetic according to Modification 4 of the embodiment of the invention, where (a) is a perspective view of a container in a state in which split molds are united, (b) is a perspective view showing an accommodation step and a compression step, (c) is a perspective view showing a taking-out step of a multicolored cosmetic bulk, (d) is a front view showing a cutting step of the multicolored cosmetic bulk, (e) is a view showing a loading step of the multicolored solid cosmetic, and (f) is a view showing a pressing step of the multicolored solid cosmetic.

As shown in (a) of FIG. 9, a container 400 used in the method for producing a multicolored solid cosmetic according to Modification 4 includes a first split mold 410 and a second split mold 420 each having a hemispherical cavity. As shown in (b) of FIG. 9, the two or more multicolored rod-shaped molded bodies 12 are disposed in the first split mold 410 (or the second split mold 420) such that the central axes B of the two or more multicolored rod-shaped molded bodies 12 are parallel to each other, and the first split mold 410 and the second split mold 420 are united and closed to compress the two or more multicolored rod-shaped molded bodies 12 into a spherical shape in the container 400. As shown in (c) of FIG. 9, the first split mold 410 and the second split mold 420 are separated, and the multicolored cosmetic bulk 10 molded into a spherical shape is taken out from the container 400. As shown in (d) of FIG. 9, the multicolored rod-shaped molded body 12 in the spherical shape is cut in a direction orthogonal to the central axis B of the rod-shaped molded body 10 in a state of being placed on a placement surface (not shown) to obtain the multicolored solid cosmetic 1. As shown in (e) of FIG. 9, the multicolored solid cosmetic 1 is loaded into the cosmetic dish 2, and as shown in (f) of FIG. 9, the multicolored solid cosmetic 1 is subjected to a press process by a press device 450 from above (arrow direction).

According to Modification 4, it is also possible to provide a multicolored solid cosmetic in which solid cosmetics of a plurality of colors are loaded in a state of being directly adjacent to each other in a cosmetic dish without partition, while obtaining the effects same as those of the above-described embodiment. In particular, according to Modification 4, a multicolored solid cosmetic formed in a shape (dome shape) bulging toward a center portion can be obtained, and a degree of freedom in shape design is increased.

Although the invention has been described with reference to the above embodiment, the invention is not limited to the above embodiment, and improvements or changes can be made within the scope of the claims.

### Reference Signs List

1: multicolored solid cosmetic
2: cosmetic dish
10: multicolored cosmetic bulk
11: rod-shaped molded body
12: multicolored rod-shaped molded body
20: container
21: first split mold
21a, 21b: side wall
22: second split mold
22a, 22b: side wall
200: container
210: first split mold
210a, 210b: side wall
211: opening
220: second split mold
300: container
301: opening
310: first split mold
310a: side wall
320: second split mold
320a: side wall
330: pressing member
400: container
410: first split mold
420: second split mold
450: press device
A: longitudinal direction
B: central axis

## Claims

1. A method for producing a multicolored solid cosmetic comprising:
a rod-shaped molded body acquisition step of obtaining a multicolored rod-shaped molded body by adhering, to a periphery of a rod-shaped molded body obtained by molding a powder cosmetic into a rod shape, a powder cosmetic having a color different from a color of the rod-shaped molded body;
a compression step of obtaining, by compressing the multicolored rod-shaped molded body in an amount of two or more disposed with central axes thereof parallel to each other in a container formed by uniting two or more split molds, a multicolored cosmetic bulk having a shape corresponding to an internal shape of the container;
a taking-out step of the multicolored cosmetic bulk; and
a cutting step of cutting the multicolored cosmetic bulk taken out from the container by separating the two or more split molds in a direction orthogonal to the central axis of the rod-shaped molded body.

2. The method for producing a multicolored solid cosmetic according to claim 1, wherein
at least one end side of the container in a longitudinal direction is formed with a side wall with which a movement in the longitudinal direction of the container is restricted, and the cutting step is performed in a state in which the multicolored cosmetic bulk is placed on a placement surface.

3. The method for producing a multicolored solid cosmetic according to claim 1 or 2, wherein
the container has a first split mold and a second split mold divided along the longitudinal direction of the container, and in the compression step, the first split mold and the second split mold are united and closed to compress the two or more multicolored rod-shaped molded bodies disposed in the first split mold and the second split mold.

4. The method for producing a multicolored solid cosmetic according to claim 1 or 2, wherein
the container has a first split mold having an opening along the longitudinal direction of the container and a second split mold configured to close the opening, and in the compression step, by pressing with the second split mold from the opening of the first split mold, the two or more multicolored rod-shaped molded bodies disposed in the first split mold are compressed.

5. The method for producing a multicolored solid cosmetic according to claim 1, wherein
the container has a first split mold and a second split mold divided along a longitudinal direction of the container, one end side of the container in the longitudinal direction is formed with a side wall with which a movement in the longitudinal direction of the container is restricted, the other end side of the container in the longitudinal direction has an opening, and in the compression step, in a state in which the first split mold and the second split mold are united, the two or more multicolored rod-shaped molded bodies in the container are compressed by being pressed by a pressing member from the opening.

6. The method for producing a multicolored solid cosmetic according to claim 1, wherein
the container has a first split mold and a second split mold in which a hemispherical cavity is formed, and in the compression step, the first split mold and the second split mold are united and closed to compress the two or more multicolored rod-shaped molded bodies disposed in the first split mold and the second split mold into a spherical shape.

7. A method for producing a multicolored solid cosmetic comprising:
a rod-shaped molded body acquisition step of obtaining, in each desired color, a rod-shaped molded body formed by molding a powder cosmetic into a rod shape;
a compression step of obtaining, by compressing the rod-shaped molded body in an amount of two or more disposed with central axes thereof parallel to each other in a container formed by uniting two or more split molds, a multicolored cosmetic bulk having a shape corresponding to an internal shape of the container;
a taking-out step of the multicolored cosmetic bulk; and
a cutting step of cutting the multicolored cosmetic bulk taken out from the container by separating the two or more split molds in a direction orthogonal to the central axis of the rod-shaped molded body.

8. The method for producing a multicolored solid cosmetic according to claim 7, wherein
the cutting step is performed in a state in which the multicolored cosmetic bulk is placed on a placement surface.

## Patentansprüche

1. Verfahren zur Herstellung eines mehrfarbigen festen Kosmetikums, umfassend:
einen Schritt zur Erlangung eines stabförmigen Formkörpers des Erhaltens eines mehrfarbigen stabförmigen Formkörpers durch Anhaftenlassen - an einer Peripherie eines stabförmigen Formkörpers, der durch Formen eines Pulverkosmetikums zu einer Stabform erhalten wurde - eines Pulverkosmetikums, das eine Farbe aufweist, die sich von einer Farbe des stabförmigen Formkörpers unterscheidet;
einen Komprimierungsschritt des Erhaltens - durch Komprimieren des mehrfarbigen stabförmigen Formkörpers in einer Menge von zwei oder mehr, angeordnet mit Mittelachsen davon parallel zueinander in einem Behälter, der durch Zusammenfügen von zwei oder mehr Teilformen ausgebildet wird - eines mehrfarbigen Kosmetikumbarrens, der eine Form aufweist, die einer inneren Form des Behälters entspricht;
einen Entnahmeschritt des mehrfarbigen Kosmetikumbarrens; und
einen Schneidschritt des Schneidens des mehrfarbigen Kosmetikumbarrens, der durch Trennen der zwei oder mehr Teilformen aus dem Behälter entnommen wurde, in einer Richtung senkrecht zur Mittelachse des stabförmigen Formkörpers.

2. Verfahren zur Herstellung eines mehrfarbigen festen Kosmetikums nach Anspruch 1, wobei
wenigstens eine Stirnseite des Behälters in einer Längsrichtung mit einer Seitenwand ausgebildet ist, mit der eine Bewegung in der Längsrichtung des Behälters begrenzt wird, und der Schneidschritt in einem Zustand erfolgt, in dem der mehrfarbige Kosmetikumbarren auf einer Auflagefläche aufliegt.

3. Verfahren zur Herstellung eines mehrfarbigen festen Kosmetikums nach Anspruch 1 oder 2, wobei
der Behälter eine erste Teilform und eine zweite Teilform aufweist, die entlang der Längsrichtung des Behälters geteilt sind, und in dem Komprimierungsschritt die erste Teilform und die zweite Teilform zusammengefügt und verschlossen werden, um die zwei oder mehr mehrfarbigen stabförmigen Formkörper, die in der ersten Teilform und der zweiten Teilform angeordnet sind, zu komprimieren.

4. Verfahren zur Herstellung eines mehrfarbigen festen Kosmetikums nach Anspruch 1 oder 2, wobei
der Behälter eine erste Teilform, die eine Öffnung entlang der Längsrichtung des Behälters aufweist, und eine zweite Teilform, die dazu ausgestaltet ist, die Öffnung zu verschließen, aufweist und in dem Komprimierungsschritt durch Drücken mit der zweiten Teilform von der Öffnung der ersten Teilform her die zwei oder mehr mehrfarbigen stabförmigen Formkörper, die in der ersten Teilform angeordnet sind, komprimiert werden.

5. Verfahren zur Herstellung eines mehrfarbigen festen Kosmetikums nach Anspruch 1, wobei
der Behälter eine erste Teilform und eine zweite Teilform aufweist, die entlang einer Längsrichtung des Behälters geteilt sind, eine Stirnseite des Behälters in der Längsrichtung mit einer Seitenwand ausgebildet ist, mit der eine Bewegung in der Längsrichtung des Behälters begrenzt wird, die andere Stirnseite des Behälters in der Längsrichtung eine Öffnung aufweist und in dem Komprimierungsschritt in einem Zustand, in dem die erste Teilform und die zweite Teilform zusammengefügt sind, die zwei oder mehr mehrfarbigen stabförmigen Formkörper in dem Behälter komprimiert werden, indem durch ein Druckausübungselement von der Öffnung her Druck auf sie ausgeübt wird.

6. Verfahren zur Herstellung eines mehrfarbigen festen Kosmetikums nach Anspruch 1, wobei
der Behälter eine erste Teilform und eine zweite Teilform aufweist, in denen ein halbkugelförmiger Hohlraum ausgebildet ist, und in dem Komprimierungsschritt die erste Teilform und die zweite Teilform zusammengefügt und verschlossen werden, um die zwei oder mehr mehrfarbigen stabförmigen Formkörper, die in der ersten Teilform und der zweiten Teilform angeordnet sind, zu einer Kugelform zu komprimieren.

7. Verfahren zur Herstellung eines mehrfarbigen festen Kosmetikums, umfassend:
einen Schritt zur Erlangung eines stabförmigen Formkörpers des Erhaltens - in jeder gewünschten Farbe - eines stabförmigen Formkörpers, der durch Formen eines Pulverkosmetikums zu einer Stabform ausgebildet wird;
einen Komprimierungsschritt des Erhaltens - durch Komprimieren des stabförmigen Formkörpers in einer Menge von zwei oder mehr, angeordnet mit Mittelachsen davon parallel zueinander in einem Behälter, der durch Zusammenfügen von zwei oder mehr Teilformen ausgebildet wird - eines mehrfarbigen Kosmetikumbarrens, der eine Form aufweist, die einer inneren Form des Behälters entspricht;
einen Entnahmeschritt des mehrfarbigen Kosmetikumbarrens; und
einen Schneidschritt des Schneidens des mehrfarbigen Kosmetikumbarrens, der durch Trennen der zwei oder mehr Teilformen aus dem Behälter entnommen wurde, in einer Richtung senkrecht zur Mittelachse des stabförmigen Formkörpers.

8. Verfahren zur Herstellung eines mehrfarbigen festen Kosmetikums nach Anspruch 7, wobei
der Schneidschritt in einem Zustand erfolgt, in dem der mehrfarbige Kosmetikumbarren auf einer Auflagefläche aufliegt.

## Revendications

1. Procédé de production d'un produit cosmétique solide multicolore comprenant :
une étape d'acquisition de corps moulé en forme de bâtonnet consistant à obtenir un corps moulé multicolore en forme de bâtonnet par adhérence, à la périphérie d'un corps moulé en forme de bâtonnet obtenu par moulage d'un produit cosmétique en poudre en une forme de bâtonnet, d'un produit cosmétique en poudre ayant une couleur différente d'une couleur du corps moulé en forme de bâtonnet ;
une étape de compression consistant à obtenir, par compression d'au moins deux corps moulés en forme de bâtonnets disposés avec leurs axes centraux parallèles entre eux dans un contenant formé par l'assemblage d'au moins deux moules divisés, une masse cosmétique multicolore ayant une forme correspond à une forme interne du contenant ;
une étape de retrait de la masse cosmétique multicolore ; et
une étape de découpe consistant à découper la masse cosmétique multicolore retirée du contenant en séparant les au moins deux moules divisés dans une direction orthogonale à l'axe central du corps moulé en forme de bâtonnet.

2. Procédé de production d'un produit cosmétique solide multicolore selon la revendication 1, dans lequel
au moins une extrémité du contenant dans une direction longitudinal est dotée d'une paroi latérale qui limite un déplacement dans la direction longitudinale du contenant, et l'étape de découpe est réalisée dans un état dans lequel la masse cosmétique multicolore est placée sur une surface de placement.

3. Procédé de production d'un produit cosmétique solide multicolore selon la revendication 1 ou 2, dans lequel
le contenant a un premier moule divisé et un deuxième moule divisé le long de la direction longitudinale du contenant, et à l'étape de compression, le premier moule divisé et le deuxième moule divisé sont assemblés et fermés afin de comprimer les au moins deux corps moulés multicolores en forme de bâtonnets disposés dans le premier moule divisé et le deuxième moule divisé.

4. Procédé de production d'un produit cosmétique solide multicolore selon la revendication 1 ou 2, dans lequel
le contenant a un premier moule divisé ayant une ouverture le long de la direction longitudinale du contenant et un deuxième moule divisé conçu pour fermer l'ouverture, et à l'étape de compression, par pression avec le deuxième moule divisé depuis l'ouverture du premier moule divisé, les au moins deux corps moulés multicolores en forme de bâtonnets disposés dans le premier moule divisé sont comprimés.

5. Procédé de production d'un produit cosmétique solide multicolore selon la revendication 1, dans lequel
le contenant a un premier moule divisé et un deuxième moule divisé le long d'une direction longitudinale du contenant, une extrémité du contenant dans la direction longitudinale est dotée d'une paroi latérale qui limite un déplacement dans la direction longitudinale du contenant, l'autre extrémité du contenant dans la direction longitudinale a une ouverture, et à l'étape de compression, dans un état dans lequel le premier moule divisé et le deuxième moule divisé sont assemblés, les au moins deux corps moulés multicolores en forme de bâtonnets dans le contenant sont comprimés en étant pressés par un élément de pression depuis l'ouverture.

6. Procédé de production d'un produit cosmétique solide multicolore selon la revendication 1, dans lequel
le contenant a un premier moule divisé et un deuxième moule divisé dans lesquels une cavité hémisphérique est formée, et à l'étape de compression, le premier moule divisé et le deuxième moule divisé sont assemblés et fermés afin de comprimer au moins deux corps moulés multicolores en forme de bâtonnets disposés dans le premier moule divisé et le deuxième moule divisé en une forme sphérique.

7. Procédé de production d'un produit cosmétique solide multicolore comprenant :
une étape d'acquisition de corps moulé en forme de bâtonnets consistant à obtenir, pour chaque couleur souhaitée, un corps moulé en forme de bâtonnet formé par moulage d'une poudre cosmétique en une forme de bâtonnet ;
une étape de compression consistant à obtenir, par compression d'au moins deux corps moulés en forme de bâtonnets disposés avec leurs axes centraux parallèles entre eux dans un contenant formé par l'assemblage d'au moins deux moules divisés, une masse cosmétique multicolore ayant une forme correspond à une forme interne du contenant ;
une étape de retrait de la masse cosmétique multicolore ; et
une étape de découpe consistant à découper la masse cosmétique multicolore retirée du contenant en séparant les au moins deux moules divisés dans une direction orthogonale à l'axe central du corps moulé en forme de bâtonnet.

8. Procédé de production d'un produit cosmétique solide multicolore selon la revendication 7, dans lequel
l'étape de découpe est réalisée lorsque la masse cosmétique multicolore est placée sur une surface de placement.
